# EUROPEAN PATENT APPLICATION

(11) **EP 4 234 575 A1**
(43) Date of publication of application: **30.08.2023**
(21) Application number: 21883265.7
(22) Date of filing: 21.10.2021
(51) Int. Cl.: C07K 14/705, C12N 5/0783, A61K 38/00, A61K 35/17, A61P 37/00, A61P 35/00, A61P 25/16, A61P 25/28, C12Q 1/6883, G01N 33/574

(54) **CELL SURFACE ANTIGEN OF ACTIVATED IMMUNE CELL AND VARIOUS USES THEREOF**

(30) Priority: 22.10.2020 KR 20200137432
(71) Applicant: Good T Cells, Inc., Seoul 03929 (KR)
(72) Inventor: KIM, Beom Seok, Seoul 03929 (KR); KIM, Jung Ho, Seoul 04136 (KR)
(74) Representative: Thomann, William John
(86) International application number: PCT/KR2021/014765
(87) International publication number: WO 2022/086197

(57) **Abstract**

The present invention relates to Lrig1 (leucine-rich and immunoglobulin-like domains 1) protein, a cell surface antigen specifically present on the surface of immune cells, and various uses thereof.

## Description

### [Technical Field]

The present invention refers to a cell surface antigen Lrig1 (leucine-rich and immunoglobulin-like domains 1), a protein that exists on the surface of activated immune cells, such as regulatory T-cells and others, which actively suppress other types of immune cells, and uses thereof.

### [Background Art]

Cancer is currently one of the diseases that cause the greatest number of deaths worldwide. Incidences of cancer are increasing in recent years due to increased life expectancy and decreased age of cancer onset. According to a statistical report by the Korean National Cancer Center in 2013, the number of cancer patients registered in the Department of Cancer Registry Statistics in 2010 was 202,053, and the numbers continue to increase to this day.

When cancer develops in a solid organ, it has traditionally been treated via surgical resection. However, in cases where the affected area is too large or when the cancer develops in a region other than a solid organ, surgical resection impractical, and hence oral or injection chemotherapy was used. Cancer cells use a protein to disrupt the cell signal transduction system by overexpression of phosphate activating proteins endogenous to the system. The anticancer drug used in chemotherapy was built and put in use to effectively bind to this protein and suppress its activity. However, this method of anticancer chemotherapy is problematic in that it can inhibit the growth of normal cells with rapid cell cycles, such as hair, and can cause various side effects including, but not limited to, dry mouth, stomatitis, nausea, vomiting, diarrhea, and neutropenia. Accordingly, efforts are being made to identify various *in vivo* molecules related to cancer and to develop drugs targeting such molecules. Efforts are also being made to enhance cancer treatment by combining these aforementioned drugs. Therefore, it can be concluded that efforts to discover and identify target molecules such as cancer-related transcription factors are crucial and highly significant.

The most important characteristic common to all normal individuals is the ability to recognize and eliminate non-self antigens while not reacting harmfully to antigenic substances that constitute the self. Such unresponsiveness to self-antigens is referred to as immunologic unresponsiveness or tolerance. Self-tolerance occurs by eliminating lymphocytes that can contain specific receptors for self-antigens, or by inactivating self-reactive functions in response to self-antigens. When problems arise in inducing or maintaining self-tolerance, immune responses against self-antigens start to occur; diseases caused by such responses are termed autoimmune diseases.

In the past, the proliferation of neuroglial cells and infiltration of immune-inflammatory cells and immune mediators observed in the pathology of neurological degenerative disease such as Alzheimer's Disease (AD), Parkinson's Disease (PD), and Amyotrophic Lateral Sclerosis (ALS) have been accepted only as non-specific secondary phenomenon resulting from the destruction of nerve cells. However, with the development of molecular biology, it became possible to identify epidermal indicators that play an important role in the immune-inflammatory mechanism. As immune-inflammatory mediators were identified and their functions revealed, there is increasing evidence to suggest that the immune-inflammatory mechanism is not merely a non-specific secondary response to nervous system damage, but can also be involved in the early stages of neurodegenerative diseases, regulating the severity of pathology and actively participating in the survival and death of neurons.

### [Disclosure]

### [Technical Problem]

One objective of the present invention is to provide a cell surface antigen for immune cells.

Another objective of the present invention is to provide a composition for preventing, improving or treating various diseases comprising immune cells, preferably immune cells with a protein encoded by the cell surface antigen of activated immune cell Lrig1 (leucine-rich and immunoglobulin-like domains 1) or the extracellular domain thereof as an active ingredient.

Another objective of the present invention is to provide a method of diagnosing various diseases comprised of an agent for measuring the Lrig1 gene, the cell surface antigen of immune cells, preferably activated immune cells, or the expression level of the protein it encodes.

Another objective of the present invention is to provide a pharmaceutical composition that comprises a bispecific antibody comprised of an antibody or antigen-binding fragment thereof that specifically binds to Lrig1, and an antibody or antigen-binding fragment thereof that specially binds to immune cell surface markers as an active ingredient for the prevention or treatment of various diseases.

Another objective of the present invention is to provide a pharmaceutical composition comprising the antibody or antigen-binding fragment thereof of the present invention or drug containing antibody-drug conjugate as an active ingredient for the prevention or treatment of various diseases.

Another objective of the present invention is to provide a method of isolating immune cells, preferably activated immune cells, by confirming changes in the expression level of the Lrig1 protein or the gene that encodes such protein.

Another objective of the present invention is to provide a method of culturing activated immune cells.

Another objective of the present invention is to provide a method for screening cell therapy products for the prevention, improvement or treatment of various diseases by confirming changes in the expression level of the Lrig1 protein or the gene that encodes such protein in immune cells.

However, the technical problem to be achieved by the present invention is not limited to the aforementioned problems, and it will be apparent to those skilled in the art that according to the gist of the present invention, the scope of the present invention extends to other problems not explicitly mentioned.

### [Technical Solution]

### 1. Cell Surface Antigens

According to one embodiment of the present invention, it relates to the cell surface antigen of immune cells of Lrig1 (leucine-rich and immunoglobulin-like domains 1).

More specifically, the cell surface antigen of the present invention is a cell surface antigen of an activated immune cell that is comprised of the Lrig1 protein or the gene that encodes such protein.

The immune cells of the present invention comprise T cells, B cells, NK cells, innate lymphoid cells, dendritic cells, neutrophils, eosinophils, basophils, macrophages, and mast cells, but is not limited thereto.

The T cells of the present invention can be effector T cells, but are not limited thereto.

The effector T cells of the present invention can be at least one of those selected from the group consisting of Th1, Th2, Th17 and Th22, but are not limited thereto.

In the present invention, "Lrig1" is a transmembrane protein consisted of 1091 amino acids that can exist on the surface of activated immune cells, such as various immune cells with activated inhibitory activity. Lrig1 consists of leucine-rich repeats (LRR) in the extracellular or lumen side, 3 immunoglobulin-like domains, transmembrane sequences, and a cytoplasmic tail. The LRIG gene family includes LRIG1, LRIG2 and LRIG3, and the amino acids are highly conserved within the family. The LRIG1 gene is highly expressed in normal skin, and can regulate the proliferation of epithelial stem cells by expressing it in basal and hair follicle cells. Therefore, it plays a crucial role in maintaining the homeostasis of the epidermis, and in its absence can develop into psoriasis or skin cancer. It has been reported that when chromosome 3p14.3 - where LRIG1 is located - is cut, there is a potential of developing into cancer cells. In fact, LRIG1 expression is greatly reduced in renal cell carcinoma and cutaneous squamous cell carcinoma.

The Lrig1 protein can be a human or mouse protein for the purpose of the present invention, but is not limited thereto.

The Lrig1 protein of the present invention can be a human-derived polypeptide (represented by SEQ ID NO: 1) or a mouse-derived polypeptide (represented by SEQ ID NO: 3), but is not limited thereto.

The Lrig1 protein (represented by SEQ ID NO: 1) of the present invention can be encoded by the polynucleotide (represented by SEQ ID NO: 2), but is not limited thereto.

The Lrig1 protein (represented by SEQ ID NO: 3) of the present invention can be encoded by the polynucleotide (represented by SEQ ID NO: 4), but is not limited thereto.

The Lrig1 of the present invention is an antigen that is specifically present on the surfaces of immune cells with activated suppressive functions against other types of immune cells, such as effector T cells. Preferably, immune cells expressing Lrig1 can exert the same inhibitory effect as regulatory T cells (Treg), but is not limited thereto.

The "Th1 cell" herein is a type of CD4+ cell, and plays a crucial role in the adaptive immune system. More specifically, it helps the activities of other immune cells by releasing cytokines that change the behavior of target cells. For the purpose of the present invention, when Lrig1 is expressed on the surface of Th1 cells, Th1 cells can escape from their intrinsic function and activate suppressor functions to behave like regulatory T cells and suppress other effector T cells, but is not limited thereto.

The "Th2 cell" herein refers to a subgroup of CD4+ T cells that secrete interleukin (IL)4, IL-5, IL-5, IL-13 due to stimulation, interferon γ that secretes Th1 helper T cells, and helper T cells that do not secrete cytokines such as IL-2. For the purpose of the present invention, when Lrig1 is expressed on the surface of Th2 cells, Th2 cells can escape from their intrinsic function and activate suppressor functions to behave like regulatory T cells and suppress other effector T cells, but is not limited thereto.

The "Th17 cell" herein refers to the population of pro-inflammatory helper T cells that produce interleukin 17. Such Th17 cells play a crucial role in adaptive immunity while protecting the body from pathogenic organisms. These Th17 cells can, like other helper T cells, be involved in B cell reinforcement in response to pathogenic organisms through mechanisms such as CXCL13 chemokine signaling. The activity of Th17 cells can be conducive to antibody formation. In particular, the Th17 cells have plasticity depending on the cytokine microenvironment and inflammatory conditions, and thus their phenotypes and functions can change.

The "Th 17 cell" herein are characterized at the molecular level by the generation of individual profiles of operational factor cytokines, IL-17A, IL-17F, IL-26, IL-22, IL-21, and TNFα, and rely on IL-23 for its development, survival and proliferation. These cytokines activate different types of cells such as keratinocytes, causing their hyperproliferation and additional production of proinflammatory cytokines, chemokines, and antibacterial peptides, which in turn recruit and activate other immune cells in inflammatory skin, resulting in amplification of inflammatory reactions. Furthermore, IL-17A and IL-17F cause the autocrine regulation of IL-17 production, which is considered to promote and maintain Th17 cell differentiation (Wei et al. 2007, J Biol. Chem., September 20). IL-17 also induces the upregulation of CCL20, the ligand of the receptor specific to Th17 cells in substrate cells, enabling further cells to lead to the inflammatory tissue. The signaling pathway of pure CD4 T cell differentiation into Th17 cell requires a combination of TGFb-1 with II-21, IL-1b and IL-23 or IL-1b, IL-23, and IL-6, which promotes Th17 cell differentiation, followed by inducing the expression of retinoid-related orphan receptor (RORC) and retinoid acid-related orphan receptor alpha (RORA), 2 transcription factors that upregulate the expression of IL-17A and II-17F (Chung Y et al. Critical regulation of early Th17 cell differentiation by interleukin-1 signaling. Immunity 2009;30:576-87, Veldhoen M, Hocking RJ, Akins CJ, Locksley RM, Stockinger B. and Immunity 2006 Feb; 24(2): 178-89). For the purpose of the present invention, when Lrig1 is expressed on the surface of Th17 cells, Th17 cells can escape from their intrinsic function and activate suppressor functions to behave like regulatory T cells and suppress other effector T cells, but is not limited thereto.

The "Th22 cell" herein refers to a subset of helper T cells that characteristically functions to produce IL-22. In particular, Th22 cells have a characteristic that allows them to specifically express IL-22 in the absence of IL-17 and INF-gamma. For the purpose of the present invention, when Lrig1 is expressed on the surface of Th22 cells, Th22 cells can escape from their intrinsic function and activate suppressor functions to behave like regulatory T cells and suppress other effector T cells, but is not limited thereto.

The "cell surface antigen" herein refers to a molecule present on the surface of a cell, which can be used as a molecule to activate receptors to specifically identify a cell or exhibit cell-specific functions. Examples of such cell surface antigens include various cell surface antigen clusters (cluster of differentiation; CD). The cell surface antigen of the present invention can be present alone or in combination with CD or alone in immune cells with activated inhibitory functions, such as effective T cells, but is not limited thereto.

### 2. Cell Therapy

In the (2) cell therapy of the present invention, contents related to immune cells and Lrig1 protein are the same as those described in (1) cell surface antigen, and thus the description thereof is omitted below.

The "cell therapy" herein refers to a living cell used in a treatment method that is directly injected into the patient. It thus refers to a drug manufactured by physical, chemical, or biological manipulation, such as culturing, proliferating, or screening living autologous cells, homologous cells, or heterogenous cells *in vitro.*

The "prevention" herein means a decrease in the degree of occurrence of pathological cells or damage or loss of cell in animals. Prevention can be either complete or partial. In this case, it can mean a phenomenon in which the occurrence of pathological cells or abnormal immune function in the subject is reduced in comparison to when the composition for preventing and treating various diseases described below is not used.

The "treatment" herein refers to any action that clinically intervenes to change the natural process of a target or cell to be treated, and can be performed during or to prevent a clinical pathological state. The desired therapeutic effects can comprise the prevention of the occurrence or recurrence of the disease, alleviation of the symptoms, reduction of any direct or indirect pathological consequences of the disease, prevention of metastasis, reduction of the rate of disease progression, mitigating or temporarily alleviating disease conditions, or improving prognosis. Thus, the treatment can be interpreted as encompassing all activities in which symptoms of various diseases described below are improved or cured by the composition.

The cells of the present invention can be administered at any cell/kg dosage between 1×10⁷ to 1×10⁸, 1×10⁸ to 2×10⁸, 2×10⁸ to 4×10⁸, 4×10⁸ to 6×10⁸, 6×10⁸ to 8×10⁸, 8×10⁸ to 1×10⁹, 1×10⁹ to 2×10⁹, 2×10⁹ to 4×10⁹, 4×10⁹ to 1×10¹⁰, 2×10⁸ to 6×10⁸, 6×10⁸ to 1×10⁹, 1×10⁸ to 2×10⁸, 2×10⁸ to 2×10⁹, 1×10⁷ to 1×10⁸, 1×10⁸ to 1×10⁹, 1×10⁹ to 1×10¹⁰, or 1×10⁷ to 1×10⁹, but is not limited thereto.

The pharmaceutical composition of the present invention can be in the form of capsules, tablets, granules, injections, ointments, powders or beverages, and the pharmaceutical composition can be characteristically intended for humans.

The pharmaceutical composition of the present invention is not limited thereto, but can be formulated in the form of oral formulations such as powders, granules, capsules, tablets, aqueous suspensions, external preparations, suppositories and sterile injection solutions according to respective conventional methods. The pharmaceutical composition of the present invention can comprise a pharmaceutically acceptable carrier. Pharmaceutically acceptable carriers can include binders, lubricants, disintegrants, excipients, solubilizers, dispersants, stabilizers, suspending agents, pigments and flavors, etc. for oral administration. In the case of injections, buffers, preservatives, non-converting agents, solubilizing agents, presenting agent, stabilizing agent, and the like can be mixed and used. In the case of local administration, base agent, excipient, lubricant, preservative and the like can be used. The formulation of the pharmaceutical composition of the present invention can be diversely prepared by mixing with the aforementioned pharmaceutically acceptable carrier. For example, the composition can be prepared in the form of tablets, troches, capsules, elixirs, suspensions, syrups, wafers, etc., for oral administrations and in the case of injections, it can be prepared in unit dosage ampoules or multiple dosage forms. In addition, it can be formulated into solutions, suspensions, tablets, capsules, sustained-release preparations, and the like.

On the other hand, examples of carriers, excipients and diluents suitable for formulation include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, tale, magnesium stearate or mineral oil, and the like. Additionally, fillers, anti-agglomerating agents, lubricants, wetting agents, flavoring agents, emulsifiers, preservatives, and the like can be further included.

The route of administration of the pharmaceutical composition of the present invention can include, but is not limited to, oral, intravenous, intramuscular, intraarterial, intramedullary, intrathecal, intracardiac, transdermal, subcutaneous, intraperitoneal, intranasal, intestinal, topical, sublingual or rectal administration, such as oral or parenteral administration.

The "parenteral" herein includes subcutaneous, intradermal, intravenous, intramuscular, intraarticular, intrasynovial, intrasternal, intrathecal, intralesional and intracranial injection or infusion techniques. For the purpose of the present invention, the pharmaceutical composition can be administered by direct injection to the kidney, but is not limited thereto.

The pharmaceutical composition of the present invention depends on various factors including the activity of the specific compound used, age, body weight, general health, sex, diet, administration time, route of administration, excretion rate, drug combination and severity of the specific disease to be prevented or treated. The dosage of the pharmaceutical composition can be variably selected depending on the patient's condition, body weight, disease severity, drug type, administration route and period, but can be appropriately selected by those skilled in the art. It can be administered at 0.0001 to 50 mg/kg or 0.001 to 50mg/kg per day. The pharmaceutical composition can be administered once a day, or can be administered across several divided dosages. The aforementioned dosages are not intended to limit the scope of the present invention in any way. The pharmaceutical composition according to the present invention can be formulated into a pill, dragee, capsule, liquid, gel, syrup, slurry, or suspension.

### 2-1. Pharmaceutical Composition for Preventing or Treating Immune-Related Diseases

In another embodiment, the present invention provides a pharmaceutical composition that is comprised of immune cells with the Lrig1 protein or the gene that encodes such protein as an active ingredient for the prevention or treatment of immune-related diseases.

The immune cells with Lrig1 protein or the gene that encodes such protein in the present invention can escape from their intrinsic function and have excellent suppressive ability against other effector T cells, so that immune-related diseases can be effectively prevented or treated.

The "immune-related disease" herein is a disease induced by excessive activation and expression of various immune cells and inflammatory cells. Examples include autoimmune disease, graft-versus-host disease, organ transplant rejection, asthma, atopy, or acute or chronic inflammatory diseases, etc., but is not limited thereto.

Additionally, the "autoimmune disease" herein can be at least one of those selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barre syndrome, chronic thyroiditis, multiple sclerosis, multiple myositis, ankylosing spondylitis, fibroblast and nodular multiple arteritis, but is not limited thereto.

### 2-2. Pharmaceutical Composition for Preventing or Treating Cancer

In another embodiment, the present invention provides a pharmaceutical composition that is comprised of immune cells with the Lrig1 protein or the gene that encodes such protein as an active ingredient for the prevention or treatment of cancer.

The immune cells with Lrig1 protein or the gene that encodes such protein in the present invention can escape from their intrinsic function and have regulate effector T cell activity to effectively suppress the growth of various cancer cells.

The "cancer" herein refers to a physiological state typically characterized by unregulated cell growth in mammals. The cancer subject to the prevention, improvement or treatment of the present invention can be, for example, a solid tumor formed by abnormal cell growth in a solid organ. Depending on the site of the solid organ, the cancer can be at least one of those selected from the group consisting of gastric cancer, liver cancer, glioblastoma, ovarian cancer, colorectal cancer, head and neck cancer, bladder cancer, renal cell cancer, breast cancer, metastasis cancer, prostate cancer, pancreatic cancer, melanoma and lung cancer, and preferably melanoma or colon cancer, but it is not limited thereto.

### 2-3. Pharmaceutical Composition for Preventing or Treating Neurodegenerative or Neuroinflammatory diseases

In another embodiment, the present invention provides a pharmaceutical composition that is comprised of immune cells with the Lrig1 protein or the gene that encodes such protein as an active ingredient for the prevention or treatment of neurodegenerative or neuroinflammatory diseases.

The immune cells with Lrig1 protein or the gene that encodes such protein in the present invention can effectively prevent or treat neurodegenerative or neuroinflammatory diseases due to their excellent ability to suppress other effector T cells.

The "neurodegenerative or neuroinflammatory disease" herein includes, but is not limited to, stroke, dementia, Alzheimer's disease, Parkinson's disease, Huntington's disease, Niemann-Pick disease, prion disease, Creutzfeldt-Jakob disease, frontotemporal dementia, Lewy dementia, Amyotrophic lateral sclerosis (ALS), paraneoplastic syndrome, corticobasal degeneration, multiple system atrophy disease, progressive supranuclear palsy, autoimmune disease of the nervous system, spinocerebellar ataxia, inflammatory and neuropathic pain, cerebrovascular disease, spinal cord injury, and tauopathy.

### 3. Diagnostic Composition

In the (3) diagnostic composition of the present invention, contents related to immune cells and Lrig1 protein are the same as those described in (1) cell surface antigen, and thus the description thereof is omitted below.

### 3-1. Composition for Diagnosis of Immune-Related Diseases

In another embodiment, the present invention provides a diagnostic composition for immune-related diseases that is comprised of an agent for measuring the expression levels of the Lrig1 protein on the surface of immune cells or of the gene that encodes such protein.

The agent for measuring the expression levels of the Lrig1 protein is not explicitly limited, but can include antibodies that specifically bind to the protein, oligopeptides, ligands, peptide nucleic acids (PNA) and aptamers.

The "antibody" herein refers to a substance that specifically binds to an antigen and causes an antigen-antibody reaction. For the purpose of the invention, antibodies refer to those that specifically bind to the Lrig1 protein. The antibody of the present invention comprises both polyclonal antibodies, monoclonal antibodies, and recombinant antibodies. Such antibodies can be readily prepared using techniques widely known in the art. For example, specifically antibodies can be produced by a method widely known in the art, which includes the process of injecting antigens of the biomarker protein into animals and collecting their blood to obtain serum containing antibodies. Such specifically antibodies can be prepared from any animal such as goat, rabbit, sheep, monkey, horse, pig, cow, dog, and the like. Additionally, monoclonal antibodies can be prepared by the hybridoma method well known in the art (see Kohler and Milstein (1976) European Journal of Immunology 6:511-519), or the phage antibody library technique (see Clackson et al., Nature, 352:624-628, 1991; Marks et al., J. Mol. Biol., 222:58, 1-597, 1991). Antibodies prepared by the aforementioned methods can be isolated and purified using methods such as gel electrophoresis, dialysis, salt precipitation, ion exchange chromatography, and affinity chromatography. Additionally, the antibody of the present invention includes not only complete forms with 2 light chains and 2 heavy chains, but also functional fragments of the antibody molecule. A functional fragment of an antibody molecule means a fragment with at least an antigen-binding function, and includes Fab, F(ab'), F(ab')2, and Fv.

The "PNA (Peptide Nucleic Acid)" herein refers to an artificially synthesized polymer similar to DNA and RNA, and was first introduced in 1991 by Professors Nielsen, Egholm, Berg and Buchardt at the University of Copenhagen, Denmark. Whereas, DNA has a phosphate-ribose backbone, PNA has a repeated N-(2-aminoethyl)-glycine backbone linked by peptides, which greatly increases the binding force and stability to DNA or RNA, and is thus used in molecular biology, diagnostic assays, and antisense therapies. PNA is thoroughly described in the literature by Nielsen PE, Egholm M, Berg RH, Buchardt O in "Sequence-selective recognition of DNA by strand displacement with a thymine-substituted polyamide" (December 1991) [Science 254 (5037): 1497-1500].

The "aptamer" herein is an oligonucleic acid or peptide molecule, and a general description of aptamers are thoroughly described in the literature [Bock LC et al., Nature 355(6360):5646(1992); Hoppe-Seyler F, Butz K "Peptide aptamers: powerful new tools for molecular medicine". J Mol Med. 78(8):42630(2000); Cohen BA, Colas P, Brent R. "an artificial cell-cycle inhibitor isolated from a combinatorial library." Proc Natl Acad Sci USA. 95(24): 142727(1998)].

The agent for measuring the expression levels of the gene encoding the Lrig1 protein (i.e., LRIG1 gene) can comprise at least one selected from the group consisting of primers, proves, and antisense nucleotides that specifically bind to the gene.

The "primer" herein is a fragment that recognizes a target gene sequence. It includes a forward and reverse primer pair, but preferably provides analysis results with specificity and sensitivity. High specificity can be imparted when the primers amplify only the target gene sequence containing complementary primary binding sites while not causing non-specific amplification because the nucleic acid sequence of the primer is inconsistent with the non-target sequence present in the sample.

The "probe" herein refers to a substance that can specifically bind to a target substance to be detected in a sample, and a substance that can specifically confirm the presence of a target substance in a sample through binding. Given probes are commonly used in the art, the type of probe is not limited but preferably can be PNA (peptide nucleic acid), LNA (locked nucleic acid), peptide, polypeptide, protein, RNA, or DNA, with PNA being the most preferred. More specifically, the probe is a biomaterial that includes those derived from or similar to those from living organisms or those manufactured in vitro. That can include enzymes, proteins, antibodies, microorganisms, animal and plant cells and organs, nerve cells, DNA, and RNA. DNA can include cDNA, genomic DNA and oligonucleotides, while RNA includes genomic RNA, mRNA, oligonucleotides, while proteins include antibodies, antigens, enzymes, peptides, and the like.

The "LNA (locked nucleic acids)" herein refers to a nucleic acid analog containing a 2'-O, 4'-C methylene bridge [J Weiler, J Hunziker and J Hall Gene Therapy (2006) 13, 496.502]. LNA nucleosides contain the common nucleic acid bases of DNA and RNA, and can base pair according to the Watson-Crick base pairing rules. However, because of the locking of molecules due to the methylene bridge, LNA is unable to form an ideal shape in the Watson-Crick base pairing. When LNAs are included in the oligonucleotides of DNA or RNA, LNAs can more rapidly pair with complementary nucleotide chains to increase the stability of the double helix.

The "antisense" herein refers to oligomers with a sequence of nucleotide bases and an inter-subunit backbone, in which the antisense oligomer is hybridized with a target sequence in RNA by Watson-Crick base pair formation, allowing the formation of mRNA and RNA:oligomer heteroduplex in the target sequence. Oligomers can have a sequence that has exactly or near complementarity to the target sequence.

Information regarding the Lrig1 protein or the LRIG1 gene that encodes such protein is known, and thus those skilled in the art can readily design primers, probes or antisense nucleotides that specifically bind to the gene that encodes the protein based on such information.

The "immune-related disease" herein is a disease induced by excessive activation and expression of various immune cells and inflammatory cells. Examples comprise autoimmune disease, graft-versus-host disease, organ transplant rejection, asthma, atopy, or acute or chronic inflammatory diseases, etc., but is not limited thereto.

Additionally, the "autoimmune disease" herein can be at least one of those selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barre syndrome, chronic thyroiditis, multiple sclerosis, multiple myositis, ankylosing spondylitis, fibroblast and nodular multiple arteritis, but is not limited thereto.

### 3-2. Composition for Diagnosis of Cancer

In another embodiment, the present invention provides a diagnostic composition for cancer that is comprised of an agent for measuring the expression levels of the Lrig1 protein on the surface of immune cells or of the gene that encodes such protein.

In the (3-2) composition for diagnosis of cancer of the present invention, contents related to an agent for measuring the expression levels of the protein or the gene that encodes it are the same as those described in (3-1) composition for diagnosis of immune-related diseases, and thus the description thereof is omitted below.

The "cancer" herein refers to a physiological state typically characterized by unregulated cell growth in mammals. The cancer subject to the prevention, improvement or treatment of the present invention can be, for example, a solid tumor formed by abnormal cell growth in a solid organ. Depending on the site of the solid organ, the cancer can be at least one of those selected from the group consisting of gastric cancer, liver cancer, glioblastoma, ovarian cancer, colorectal cancer, head and neck cancer, bladder cancer, renal cell cancer, breast cancer, metastasis cancer, prostate cancer, pancreatic cancer, melanoma and lung cancer, and preferably melanoma or colon cancer, but it is not limited thereto.

### 3-3. Composition for Diagnosis of Neurodegenerative or Neuroinflammatory Disease

In another embodiment, the present invention provides a diagnostic composition for neurodegenerative or neuroinflammatory diseases that is comprised of an agent for measuring the expression levels of the Lrig1 protein on the surface of immune cells or of the gene that encodes such protein.

In the (3-3) composition for diagnosis of neurodegenerative or neuroinflammatory disease of the present invention, contents related to an agent for measuring the expression levels of the protein or the gene that encodes it are the same as those described in (3-1) composition for diagnosis of immune-related diseases, and thus the description thereof is omitted below.

The "neurodegenerative or neuroinflammatory disease" herein comprises, but is not limited to, stroke, dementia, Alzheimer's disease, Parkinson's disease, Huntington's disease, Niemann-Pick disease, prion disease, Creutzfeldt-Jakob disease, frontotemporal dementia, Lewy dementia, Amyotrophic lateral sclerosis (ALS), paraneoplastic syndrome, corticobasal degeneration, multiple system atrophy disease, progressive supranuclear palsy, autoimmune disease of the nervous system, spinocerebellar ataxia, inflammatory and neuropathic pain, cerebrovascular disease, spinal cord injury, and tauopathy.

### 4. Diagnostic Kit

In the (4) diagnostic kit of the present invention, contents related to immune cells and the Lrig1 protein are the same as those described in (1) cell surface antigens, and thus the description thereof is omitted below.

### 4-1. Diagnostic Kit for Immune-Related Diseases

In another embodiment, the present invention provides a diagnostic kit for immune-related diseases that is comprised of a diagnostic composition for immune-related diseases.

In the present invention, the kit can be a RT-PCR kit, DNA Chip kit, ELISA kit, protein Chip kit, rapid kit or MRM (multiple reaction monitoring) kit, but is not limited thereto.

The kit of the present invention can further include one or more other component compositions, solutions, or devices suitable for the analysis method.

For example, the kit of the present invention can comprise additional essential elements necessary for carrying out the reverse transcription polymerase reaction. The reverse transcription polymerase reaction contains a pair of primers specific to a gene encoding a marker protein. The primer is a nucleotide with a sequence specific to the nucleic acid sequence of the given gene, and can have a length of about 7bp to 50bp, more preferably between 10bp and 30bp. A primer specific to the nucleic acid sequence of the control gene can also be included. Other reverse transcription polymerase reaction kits can include test tubes or other suitable container, reaction buffer (with varying pH and magnesium concentrations), deoxynucleotides (dNTPs), enzymes such as Taq-polymerase and reverse transcriptase, DNase and RNase inhibitor DEPC-water, sterilized water, and the like.

In addition, the diagnostic kit of the present invention can include essential elements necessary for carrying out DNA Chip. The DNA Chip kit can include a substrate to which cDNA or oligonucleotides corresponding to genes or fragments thereof are attached, as well as reagents, agents, and enzymes for producing fluorescently labeled probes. Additionally, the substrate can include a cDNA or oligonucleotide corresponding to a control gene or a fragment thereof.

In addition, the diagnostic kit of the present invention can include essential elements necessary for carrying out ELISA. The ELISA kit includes antibodies specific to the protein. The antibody is a monoclonal antibody, polyclonal antibody, or recombinant antibody that has high specificity and affinity for a marker protein and little cross-reactivity to other proteins. The ELISA kit can also include antibodies specific to a control protein. Other ELISA kits can include reagents capable of detecting bound antibodies such as labeled secondary antibodies, chromophores, enzymes (e.g., conjugated with antibodies), substrates thereof or those capable of binding the antibody, and the like.

### 4-2. Diagnostic Kit for Cancer

In another embodiment, the present invention provides a diagnostic kit for cancer that is comprised of a diagnostic composition for cancer.

In the (4-2) diagnostic kit for cancer of the present invention, contents related to an agent for measuring the expression levels of the protein or the gene that encodes it are the same as those described in (3-1) composition for diagnosis of immune-related diseases, while contents related to kits are the same as those described in (4-1) diagnostic kit for immune-related diseases. Thus, the description thereof is omitted below.

### 4-3. Diagnostic Kit for Neurodegenerative or Neuroinflammatory Diseases

In another embodiment, the present invention provides a diagnostic kit for neurodegenerative or neuroinflammatory diseases that is comprised of a diagnostic composition for neurodegenerative or neuroinflammatory diseases.

In the (4-3) diagnostic kit for neurodegenerative or neuroinflammatory diseases of the present invention, contents related to an agent for measuring the expression levels of the protein or the gene that encodes it are the same as those described in (3-1) composition for diagnosis of immune-related diseases, while contents related to kits are the same as those described in (4-1) diagnostic kit for immune-related diseases. Thus, the description thereof is omitted below.

### 5. Diagnosis Method

In the (5) diagnosis method of the present invention, contents related to immune cells and the Lrig1 protein are the same as those described in (1) cell surface antigens, and thus the description thereof is omitted below.

### 5-1. Methods of Providing Information for Diagnosing Immune-Related Diseases

In another embodiment, the present invention provides a method for providing information for diagnosing immune-related diseases comprised of measuring the expression level of Lrig1 protein or the gene that encodes such protein in immune cells among biological samples isolated from the objective individual.

The "objective individual" herein refers to an individual whose occurrence of the disease is uncertain and which has a high possibility of developing the disease.

The "biological sample" herein refers to any material, biological fluid, tissue or cell obtained or derived from an individual. It can include whole blood, leukocytes, peripheral blood mononuclear cells, buffy coat, plasma, and serum-containing blood, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cell, cell extract, or cerebrospinal fluid, but is not limited thereto.

In the (5-1) methods of providing information for diagnosing immune-related diseases of the present invention, contents related to an agent for measuring the expression levels of the Lrig1 protein and the gene that encodes it overlaps with what is stated in (3-1) composition for diagnosis of immune-related diseases, and thus the description thereof is omitted below.

The methods for measuring or comparing the expression levels of Lrig1 protein include protein Chip analysis, immunoassay, ligand binding assay, MALDI-TOF (Matrix Assisted Laser Desorption/lonization Time of Flight Mass Spectrometry) analysis, SELDI-TOF (Surface Enhanced Laser Desorption/lonization Time of Flight Mass Spectrometry) analysis, radioimmunoassay, radio immunodiffusion method, Oukteroni immunodiffusion method, rocket immunoelectrophoretic, tissue immunostaining, complement fixation assay, 2D electrophoresis assay, Liquid Chromatography-Mass Spectrometry (LC-MS), LC-MS/MS (Liquid Chromatography-Mass Spectrometry/Mass Spectrometry), Western blotting, or ELISA (enzyme linked immunosorbent assay), but are not limited thereto.

The analysis methods to measure the expression level of the gene encoding Lrig1 protein to confirm the presence and expression level of the gene include reverse transcription polymerase reaction (Real-time; RT-PCR), RPA (RNase Protection Assay), Northern blotting, and DNA Chip, but is not limited thereto.

When the expression level of the Lrig1 protein of the immune cell or the gene encoding it - measured in the biological sample of the objective individual - is increased or decreased in comparison to the normal control group, the prediction of a high possibility of developing immune-related diseases can be included.

More specifically, if the expression level of the Lrig1 protein of the immune cell or the gene encoding it - measured in the biological sample of the objective individual - is decreased in comparison to the normal control group, it can be predicted that there is a high possibility of developing immune-related diseases.

Moreover, when predicting or diagnosing a high possibility of developing immune-related diseases by measuring the expression level of the Lrig1 protein or the gene encoding it - measured in the biological sample of the objective individual as aforementioned - it can further include a step of administering a drug for the disease to the objective individual.

### 6. Bispecific Antibodies

In the (6) bispecific antibodies of the present invention, contents related to immune cells and the Lrig1 protein are the same as those described in (1) cell surface antigens, and thus the description thereof is omitted below.

### 6-1. Pharmaceutical Composition for Preventing or Treating Immune-Related Diseases

In another embodiment, the present invention provides a pharmaceutical composition that is comprised of bispecific antibodies that specifically bind to Lrig1 or immune cell surface markers as an active ingredient for preventing or treating immune-related diseases.

The bispecific antibody of the present invention includes an antibody or antigen-binding fragment thereof that specifically binds to Lrig1, and an antibody or antigen-binding fragment thereof that specially binds to immune cell surface markers.

The immune cells of the present invention comprise T cells, B cells, NK cells, innate lymphoid cells, dendritic cells, neutrophils, eosinophils, basophils, macrophages, and mast cells, but is not limited thereto.

The T cells of the present invention can be effector T cells, but are not limited thereto.

The effector T cells of the present invention can be at least one of those selected from the group consisting of Th1, Th2, Th17 and Th22, but are not limited thereto.

The bispecific antibody of the present invention double targets the Lrig1 protein and surface markers specifically expressed on immune cells, and thus it specifically binds to activated effector T cells whose functions or properties can change depending on the surrounding microenvironment. Therefore, it can very effectively prevent or treat immune-related diseases because it effectively regulates not only the immune cell-specific functions but also the immunosuppressive functions of regulatory T cells,

The "bispecific antibody" herein can be provided in any suitable format, such as the format listed in the literature which is incorporated herein by reference in its entirety (see Kontermann MAbs 2012, 4(2): 182-197). For example, a bispecific antibody or bispecific antigen-binding fragment can be bispecific antibody conjugates (e.g. IgG2, F(ab')2 or CovX-body), bispecific IgG or IgG-like molecules (e.g. IgG, scFv4-Ig, IgG-scFv, scFv-IgG, DVD-Ig, IgG-sVD, sVD-IgG, or 2 in 1-lgG, mAb2, or Tandemab common LC), asymmetric bispecific IgG or IgG-like molecules (e.g. Kih IgG, kih IgG common LC, CrossMab, kih IgG-scFab, mAb-Fv, charge pairs or SEED-body), small bispecific antibody molecules (e.g. Diabodies (Db), dsDb, DART, scDb, tandAbs, tandem scFv (taFv), tandem dAb/VHH, triple body, triple head, Fab-scFv, or F(ab')2-scFv2), bispecific Fc and CH3 fusion proteins (e.g. taFv-Fc, di-diabody, scDb-CH3, scFv-Fc-scFv, HCAb-VHH, scFv-kih-Fc, or scFv-kih-CH3), or bispecific fusion proteins (e.g. scFv2-albumin, scDb-albumin, taFv-toxin, DNL-Fab4-IgG, DNL-Fab4-IgG-cytokine 2). In particular, reference Figure 2 of the literature (Kontermann MAbs 2012, 4(2): 182-19). One skilled in the art can design and prepare bispecific antibodies and bispecific antigen-binding fragments according to the present invention.

Methods for producing the bispecific antibodies in the present invention include chemical crosslinking of an antibody or antibody fragment with reductive disulfide or nonreducing thioether bond, as proposed in the literature that is incorporated herein by reference in its entirety [See: Segal and Bast, 2001. Production of Bispecific Antibodies, Current Protocols in Immunology. 14:IV:2.13:2.1-2.13.16]. For example, N-succinimidyl-3-(-2-pyridyldithio)-propionate (SPDP) can be used to chemically crosslink Fab fragments via hinge region SH- groups to generate disulfide-linked bispecific F(ab)2 heterodimers.

In addition, another method for producing the bispecific antibodies in the present invention include, as proposed in the literature [See: D.M. and Bast, B.J. 2001. Production of Bispecific Antibodies. Current Protocols in Immunology. 14:IV:2.13:1-2.13.16], the fusion of antibody-producing hybrids with polyethylene glycols, for example, to produce quadroma cells capable of secreting bispecific antibodies.

The bispecific antibodies and the bispecific antibody fragments can also be produced via recombination due to expression of a nucleic acid construct encoding a polypeptide for an antigen-binding molecule, as proposed in the two literature which is incorporated herein by reference in its entirety [See: Antibody Engineering: Methods and Protocols, Second Edition (Humana Press, 2012), at Chapter 40: Production of Bispecific Antibodies: Diabodies and Tandem scFv (Hornig and Farber-Schwarz), or French, How to make bispecific antibodies, Methods Mol. Med. 2000; 40:333-339]. For example, the DNA constructs comprising sequences that encode light and heavy chain variable domains for 2 antigen-binding domains (hence, light and heavy chain variable domains for antigen-binding domains that can bind to PD-1, and light and heavy chain variable domains for antigen-binding domains that can bind to other target proteins) and include sequences encoding suitable linkers or dimerization domains between antigen-binding domains can be generated via molecular cloning techniques. The recombinant bispecific antibodies can then be produced by expression of the conduct (e.g., in vitro) in a suitable host cell (e.g., mammalian host cell). The resulting expressed recombinant bispecific antibody can then optionally be purified.

The antibody of the present invention can be generated by an affinity maturation process that produces modified antibodies with improved affinity of the antibody for the antigen, when compared to an unmodified parent antibody. Affinity matured antibodies can be generated by procedures well described in the art, such as those proposed in the literature [See: Marks et al., Rio/Technology 10:779-783 (1992); Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-159 (1995); or Hawkins et al., J. Mol. Biol. 226:889-896 (1992)].

The "antibody" herein refers to a substance produced by the stimulation of antigens in the immune system. It can comprise, but is not limited to, those produced in vivo, recombinants, or artificially synthesized antibodies. The present invention comprises all animal antibodies, chimeric antibodies, humanized antibodies, and human antibodies. The antibody of the present invention also comprises antigen-binding fragments of antibodies with antigen-binding ability.

The "antigen-binding fragment" herein can be selected from antibody fragments that comprise more than 1 complementarity determining region, such as scFv, (scFv)2, scFv-Fc, Fab, Fab', and F(ab')2.

The antibody or antigen-binding fragment thereof that specifically binds to Lrig1 of the present invention can comprise those selected from the group consisting of:
(a) heavy chain variable region comprising heavy chain CDR1 represented by SEQ ID NO:5, heavy chain CDR2 represented by SEQ ID NO:6, and heavy chain CDR3 represented by SEQ ID NO:7;
(b) heavy chain variable region comprising heavy chain CDR1 represented by SEQ ID NO:11, heavy chain CDR2 represented by SEQ ID NO:12, and heavy chain CDR3 represented by SEQ ID NO:13;
(c) heavy chain variable region comprising heavy chain CDR1 represented by SEQ ID NO:17, heavy chain CDR2 represented by SEQ ID NO:18, and heavy chain CDR3 represented by SEQ ID NO:19;
(d) heavy chain variable region comprising heavy chain CDR1 represented by SEQ ID NO:23, heavy chain CDR2 represented by SEQ ID NO:24, and heavy chain CDR3 represented by SEQ ID NO:25;
(e) heavy chain variable region comprising heavy chain CDR1 represented by SEQ ID NO:29, heavy chain CDR2 represented by SEQ ID NO:30, and heavy chain CDR3 represented by SEQ ID NO:31;
(f) heavy chain variable region comprising heavy chain CDR1 represented by SEQ ID NO:35, heavy chain CDR2 represented by SEQ ID NO:36, and heavy chain CDR3 represented by SEQ ID NO:37;
(g) heavy chain variable region comprising heavy chain CDR1 represented by SEQ ID NO:41, heavy chain CDR2 represented by SEQ ID NO:42, and heavy chain CDR3 represented by SEQ ID NO:43;
(h) heavy chain variable region comprising heavy chain CDR1 represented by SEQ ID NO:11, heavy chain CDR2 represented by SEQ ID NO:12, and heavy chain CDR3 represented by SEQ ID NO:13;
(i) light chain variable region comprising light chain CDR1 represented by SEQ ID NO:8, light chain CDR2 represented by SEQ ID NO:9, and light chain CDR3 represented by SEQ ID NO:10;
(j) light chain variable region comprising light chain CDR1 represented by SEQ ID NO:14, light chain CDR2 represented by SEQ ID NO:15, and light chain CDR3 represented by SEQ ID NO:16;
(k) light chain variable region comprising light chain CDR1 represented by SEQ ID NO:20, light chain CDR2 represented by SEQ ID NO:21, and light chain CDR3 represented by SEQ ID NO:22;
(l) light chain variable region comprising light chain CDR1 represented by SEQ ID NO:26, light chain CDR2 represented by SEQ ID NO:27, and light chain CDR3 represented by SEQ ID NO:28;
(m) light chain variable region comprising light chain CDR1 represented by SEQ ID NO:32, light chain CDR2 represented by SEQ ID NO:33, and light chain CDR3 represented by SEQ ID NO:34;
(n) light chain variable region comprising light chain CDR1 represented by SEQ ID NO:38, light chain CDR2 represented by SEQ ID NO:39, and light chain CDR3 represented by SEQ ID NO:40;
(o) light chain variable region comprising light chain CDR1 represented by SEQ ID NO:44, light chain CDR2 represented by SEQ ID NO:45, and light chain CDR3 represented by SEQ ID NO:46;
(p) light chain variable region comprising light chain CDR1 represented by SEQ ID NO:50, light chain CDR2 represented by SEQ ID NO:51, and light chain CDR3 represented by SEQ ID NO:52.

The surface markers of immune cells of the present invention can comprise any marker that is specifically expressed on the surface of individual immune cells to allow such cells to be distinguished from other immune cells.

The "Th1 cell surface marker" herein refers to a marker that is specifically expressed on the surface of Th1 cells and can distinguish Th1 cells from other. It can include CD94, CD119 (IFNγR1), IFNγR2, CD183 (CXCR3), CD186 (CDCR6), CD191 (CCR1), CD195 (CCR5), CD212 (IL-12Rβ1), IL-12Rβ2, CD218a (IL-18Rα), IL-27Rα, CD254 (RANKL) or CD366 (TIM3), but is not limited thereto.

The "Th2 cell surface marker" herein refers to a marker that is specifically expressed on the surface of Th2 cells and can distinguish Th2 cells from other. It can include CD184 (CDCR4), CD193 (CCR3), CD194 (CCR4), CD198 (CCR8), CD294 (CRTH2), CD365 (TIM1), IL-25R (IL-17RB), IL-33R (ST2), IL-4R or TSLPR, but is not limited thereto.

The "Th17 cell surface marker" herein refers to a marker that is specifically expressed on the surface of Th17 cells and can distinguish Th17 cells from other. It can include CD121a (IL-1RI), CD126 (IL-6Rα), CD161, CD194 (CCR4), CD196 (CCR6), IL-23R, CD360 (IL-21R), CD212 (IL-12Rβ1) or TGF-βRII, but is not limited thereto.

The "Th22 cell surface marker" herein refers to a marker that is specifically expressed on the surface of Th22 cells and can distinguish Th22 cells from other. It can include CD194 (CCR4), CD196 (CCR6), CCR10, IL-6Rα, TGF-βRII or TNFRI, but is not limited thereto.

The other regions of the bispecific antibody except the heavy chain CDR regions, light chain CDR sites, heavy chain variable regions or the light chain variable regions of the present invention can include those derived from all subtypes of immunoglobulin (e.g., IgA, IgD, IgE, IgG, (lgG1, IgG2, IgG3, IgG4), IgM, etc.), such as those derived from the light chain constant region and/or heavy chain constant region of all subtypes of immunoglobulin.

The "complementarity-determining regions (CDR)" herein refer to the parts of the antibody's variable regions that gives binding specificity to the antigen.

### 6-2. Pharmaceutical Composition for Preventing or Treating Cancer

In another embodiment, the present invention provides a pharmaceutical composition that is comprised of bispecific antibodies that specifically bind to Lrig1 or immune cell surface markers as an active ingredient for preventing or treating cancer.

In the (6-2) pharmaceutical composition for preventing or treating cancer of the present invention, contents related to bispecific antibodies, antibodies or binding fragments thereof that specifically bind to Lrig1 or immune cell surface markers are the same as those described in (6-1) pharmaceutical composition for preventing or treating immune-related diseases, and thus the description thereof is omitted below.

### 6-3. Pharmaceutical Composition for Preventing or Treating Neurodegenerative or Neuroinflammatory Diseases

In another embodiment, the present invention provides a pharmaceutical composition that is comprised of bispecific antibodies that specifically bind to Lrig1 or immune cell surface markers as an active ingredient for preventing or treating neurodegenerative or neuroinflammatory diseases.

In the (6-3) pharmaceutical composition for preventing or treating neurodegenerative or neuroinflammatory diseases of the present invention, contents related to bispecific antibodies, antibodies or binding fragments thereof that specifically bind to Lrig1 or immune cell surface markers are the same as those described in (6-1) pharmaceutical composition for preventing or treating immune-related diseases, and thus the description thereof is omitted below.

### 6-4. Combined Administration

The pharmaceutical composition of the present invention can be additionally administered in combination with other therapeutic agents for immune-related diseases, cancer, neurodegenerative or neuroinflammatory diseases.

The treatment for immune-related diseases of the present invention can be immunomodulators such as cytokines, stem cell growth factors, lymphotoxins, hematopoietic factors, colony stimulating factors (CSF), interferons (IFN), erythropoietin, thrombopoietin or an anti-inflammatory agent such as a steroid or non-steroidal anti-inflammatory agent, but is not limited thereto.

The treatment for cancer of the present invention, hence the anticancer drug, of the present invention can be selected from the group consisting of nitrogen mustard, imatinib, oxaliplatin, rituximab, erlotinib, neratinib, lapatinib, gefitinib, vandetanib, nirotinib, semasanib, bosutinib , axitinib, cediranib, lestaurtinib, trastuzumab, gefitinib, bortezomib, sunitinib, carboplatin, bevacizumab, cisplatin, cetuximab, viscum album, asparaginase, tretinoin, Hydroxycarbamide, dasatinib, estramustine, gemtuzumab ozogamicin, ibritumomabtucetan, heptaplatin, methylaminolevulinic acid, amsacrine, alemtuzumab, procarbazine, alprostadil , holmium nitrate chitosan, gemcitabine, doxifluridine, pemetrexed, tegafur, capecitabine, gimeracin, oteracil, azacytidine, methotrexate, uracil, cytarabine, fluorouracil, fludagabine , enocitabine, flutamide, decitabine, mercaptopurine, thioguanine, cladribine, carmophor, raltitrexed, docetaxel, paclitaxel, irinotecan, belotecan, topotecan, vinorelbine, etoposide, bin Cristine, Vinblastine, Teniposide, Doxorubicin, Idarubicin, Epirubicin, Mitoxantrone, Mitomycin, Bleromycin, Daunorubicin, Dactinomycin, Pirarubicin, Aclarubicin, Pepro Mycin, temsirolimus, temozolomide, busulfan, ifosfamide, cyclophosphamide, melpharan, altretmin, dacarbazine, thiotepa, nimustine, chlorambucil, mitolactol, leucovorin, thre tonin, exmestane, aminoglutecimide, anagrelide, navelbine, fadrazole, tamoxifen, toremifene, testolactone, anastrozole, letrozole, vorozole, bicalutamide, lomustine or carmustine, but are not limited thereto.

The treatment for neurodegenerative or neuroinflammatory diseases of the present invention can comprise AchE inhibitors, NMDA receptor antagonists, dopamine substitutes, dopamine agonists, MAO inhibitors, small molecule therapeutic drugs like COMT inhibitors, gene therapy, monoclonal antibody therapy, immunotherapy, or iPSC, but is not limited thereto.

### 7. Bispecific Antibody-Drug Conjugates

In the (7) bispecific antibody-drug conjugates of the present invention, contents related to immune cells and Lrig1 protein are the same as those described in (1) cell surface antigen, and thus the description thereof is omitted below.

Additionally, in the (7) bispecific antibody-drug conjugates of the present invention, contents related to concomitant administration are the same as those described in (6-4) concomitant administration, and thus the description thereof is omitted below.

### 7-1. Pharmaceutical Composition for Preventing or Treating Immune-Related Diseases

In another embodiment, the present invention provides a pharmaceutical composition that is comprised of bispecific antibodies or antigen-binding fragments that specifically bind to Lrig1 or immune cell surface markers and drug containing antibody-drug conjugates as an active ingredient for preventing or treating immune-related diseases.

The "antibody-drug conjugate (ADC)" herein refers a form that chemically connects drugs and antibodies without degrading the biological activity of the antibodies and drugs. In the present invention, the antibody-drug conjugate refers to a form in which the drug is bond to an amino acid residue at the N-terminal of the heavy and/or light chain of an antibody; more specifically a form in which the drug is bound to the α-amine group at the N-terminal of the heavy and/or light chain of an antibody.

The "drug" herein is a concept that can refer to any substance with a specific biological activity in cells including DNA, RNA or peptide. The drug can take a form comprising a reactive group capable of reacting and cross-linking with an α-amine group, as well as a form that is connected to a linker containing a reactive group capable of reacting and cross-linking with an α-amine group.

The reactive group capable of reacting to and cross-linking with an α-amine group is not particularly limited as long as it is capable of cross-linking with the α-amine group at the N-terminal of the heavy and/or light chain of an antibody, and thus includes all types well known in the art to react to an amine group such as Isothiocyanate, Isocyanates, Acyl azide, NHS ester, Sulfonyl chloride, Aldehyde, Glyoxal, Epoxide, Oxirane, Carbonate, Aryl halide, Imidoester, Carbodiimide, Anhydride or Fluorophenyl ester, but is not limited thereto.

### 7-2. Pharmaceutical Composition for Preventing or Treating Cancer

In another embodiment, the present invention provides a pharmaceutical composition that is comprised of bispecific antibodies or antigen-binding fragments that specifically bind to Lrig1 or immune cell surface markers and antibody-drug conjugates, including/such as drugs as an active ingredient for preventing or treating cancer.

In the (7-2) pharmaceutical composition for preventing or treating cancer of the present invention, contents related to antibody-drug conjugates that specifically bind to Lrig1 or immune cell surface markers are the same as those described in (7-1) pharmaceutical composition for preventing or treating immune-related diseases, and thus the description thereof is omitted below.

### 7-3. Pharmaceutical Composition for Preventing or Treating Neurodegenerative or Neuroinflammatory Diseases

In another embodiment, the present invention provides a pharmaceutical composition that is comprised of bispecific antibodies or antigen-binding fragments that specifically bind to Lrig1 or immune cell surface markers and drug containing antibody-drug conjugates as an active ingredient for preventing or treating neurodegenerative or neuroinflammatory diseases.

In the (7-3) pharmaceutical composition for preventing or treating neurodegenerative or neuroinflammatory diseases of the present invention, contents related to antibody-drug conjugates that specifically bind to Lrig1 or immune cell surface markers are the same as those described in (7-1) pharmaceutical composition for preventing or treating immune-related diseases, and thus the description thereof is omitted below.

### 8. Method to Isolate Immune Cells

In another embodiment, the present invention provides a method for isolating activated immune cells.

In the (8) method to isolate immune cells of the present invention, contents related to immune cells and Lrig1 protein are the same as those described in (1) cell surface antigen, and thus the description thereof is omitted below.

The isolation method of the present invention comprises measuring the expression level of Lrig1 protein or the gene encoding it in a separate biological sample.

The "separate biological sample" herein can be biological samples isolated from healthy normal controls or individuals seeking immunotherapy. More specifically, it refers to any substance, biological fluid, tissue, or cell obtained or derived from an individual such as whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cell, cell extract, or cerebrospinal fluid, but is not limited thereto.

The isolation method of the present invention can isolate immune cells expressing Lrig1 protein or the gene encoding it, immune cells in which the expression levels of such are increased in comparison to the normal control group or immune cells in which the expression levels of such are increased in comparison to other average immune cells.

The immune cells isolated according to the isolation method of the present invention can be administered to individuals who have, or is highly likely to develop, immune-related diseases, cancer or neurodegenerative or neuroinflammatory diseases for the prevention, improvement, or treatment of the disease. Here, the individual to which the immune cells are administered can be the same as or different from the individual from which the biological sample is derived.

### 9. Method to Culture Immune Cells

In another embodiment, the present invention provides a method for isolating activated immune cells.

In the (9) method to culture immune cells of the present invention, contents related to immune cells and Lrig1 protein are the same as those described in (1) cell surface antigen, and thus the description thereof is omitted below.

The culturing method of the present invention includes isolating activated immune cells by measuring the expression level of Lrig1 protein or the gene encoding it in a separate biological sample and culturing such activated immune cells.

In the (9) method to culture immune cells of the present invention, contents related to the isolation of activated immune are the same as those described in (8) method to isolate immune cells, and thus the description thereof is omitted below.

The "culture (or culturing)" of the culturing method of the present invention refers to any in vitro cell cultures. In the present invention, cell culture mediums - which refer to all types of media used in a cell culture environment - can be used and can include amino acids, at least one carbohydrate as an energy source, trace elements, vitamins, salts and other additional ingredients (e.g., that affect the growth, productivity or product quality of the cells), but is not limited thereto.

### 10. Method for Screening Cell Therapies

In the (10) method for screening cell therapies of the present invention, contents related to immune cells and Lrig1 protein are the same as those described in (1) cell surface antigen, and thus the description thereof is omitted below.

### 10-1. Method for Screening Cell Therapies for Immune-Related Diseases

In another embodiment, the present invention provides a method for screening cell therapies for immune-related diseases.

The screening method of the present invention comprises measuring the expression level of Lrig1 protein or the gene encoding it in a separate biological sample.

In the screening method of the present invention, if the expression level of the Lrig1 protein or the gene encoding it in immune cells of isolated biological samples is increased or decreased in comparison to the control group, it can be selected as a cell therapy for immune-related diseases.

In the method for screening cell therapies of the present invention, contents related to cell therapy, biological samples, methods of measuring the expression levels of proteins or the gene encoding it, and immune-related diseases are the same as those described above, and thus the description thereof is omitted below.

### 10-2. Method for Screening Cell Therapies for Cancer

In another embodiment, the present invention provides a method for screening cell therapies for cancer.

The screening method of the present invention comprises measuring the expression level of Lrig1 protein or the gene encoding it in a separate biological sample.

In the screening method of the present invention, if the expression level of the Lrig1 protein or the gene encoding it in immune cells of isolated biological samples is increased or decreased in comparison to the control group, it can be selected as a cell therapy for cancer.

In the method for screening cell therapies of the present invention, contents related to cell therapy, biological samples, methods of measuring the expression levels of proteins or the gene encoding it, and immune-related diseases are the same as those described above, and thus the description thereof is omitted below.

### 10-3. Method for Screening Cell Therapies for Neurodegenerative or Neuroinflammatory Diseases

In another embodiment, the present invention provides a method for screening cell therapies for neurodegenerative or neuroinflammatory diseases.

The screening method of the present invention comprises measuring the expression level of Lrig1 protein or the gene encoding it in a separate biological sample.

In the screening method of the present invention, if the expression level of the Lrig1 protein or the gene encoding it in immune cells of isolated biological samples is increased or decreased in comparison to the control group, it can be selected as a cell therapy for neurodegenerative or neuroinflammatory diseases.

In the method for screening cell therapies of the present invention, contents related to cell therapy, biological samples, methods of measuring the expression levels of proteins or the gene encoding it, and immune-related diseases are the same as those described above, and thus the description thereof is omitted below.

### [Advantageous Effects]

The immune cells of the present invention have an activated inhibitory function, and the Lrig1 protein of activated immune cells can be used as a new target for immune-related diseases, cancer, or neurodegenerative or neuroinflammatory diseases.

### [Description of Drawings]

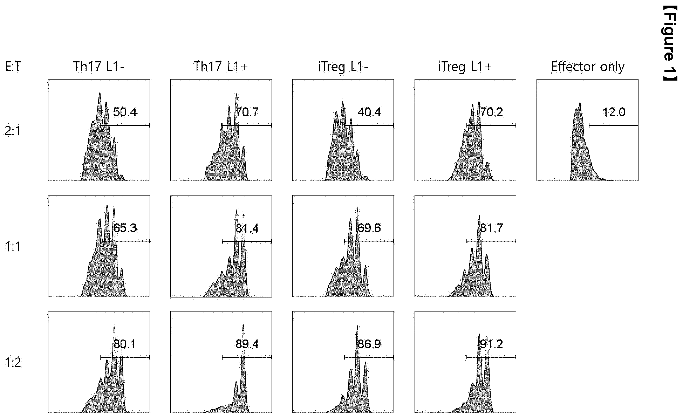

Figure 1 shows the results confirming the inhibitory effect of Th17 cells according to an embodiment of the present invention.

### [Best Mode]

The results confirming the inhibitory effect of Th17 cells according to an embodiment of the present invention is shown in Figure 1.

### [Mode For Invention]

Hereinafter, the present invention will be described in detail by following the embodiments. However, the following embodiments only illustrate the present invention and thus the content of the invention is not restricted nor limited by the following.

### [Examples]

### [Preparation Example] T cell subset cell culture

To confirm the effector T cell inhibitory effects of Th17 cells, T cell subsets Th0, Th1, Th2, Th17 and iTreg were prepared. Unlike naturally isolated nTreg, iTreg refers to cells artificially induced to differentiate in a medium containing the following composition.

T cell subtypes were induced to differentiate by first isolating naive T cells from the spleen of mice using MACS according to a conventional method. Then the components of Table 1 below were added to a RPMI1640 (Invitrogen Gibco, Grand Island, NY) nutritional medium containing 10% Fetal Bovine Serum (FBS; Hyclone, Logan, UT), and the cells were incubated in a 37°C, 5% CO₂ culture medium for 72 hours to differentiate into individual cells.

**[Table 1]**

| **Differentiated Cell** | **Composition** |
|---|---|
| Th0 | anti-CD3, anti-CD28 |
| Th1 | IL-12, anti-IL-4 antibody |
| Th2 | IL-4, anti-IFNβ |
| Th17 | IL-6, TGFβ, anti-IFNγ, anti-IL-4 |
| iTreg | IL-2, TGFβ |

### [Example] Confirmation of Effector T Cell Inhibitory Effect

The differentiated cells from the [Preparation Example] were classified into cells with high expression levels of Lrig1 (Th17 L1+ or iTreg L1+) and cells with low expression levels of Lrig1 (Th17 L1- or iTreg L1-) using the Fluorescence-Activated Cell Sorter; FACS).

Then, to confirm the effector T cell inhibitory effect, antigen presenting cells (APC) and naive CD4+ T cells were washed twice with PBS to completely remove serum. When the above pellet was dissolved with 1mL of PBS for CFSE labeling, in the case of 2×10⁷ cells, 1 mL of a 5 mM CFSE 0.25 µL mixture was added to 1 mL of PBS and mixed in a 1:1 ratio. However, when the cell count was lower than 2×10⁷, it was dissolved with 500 µL of PBS and mixed with 500 µL of the above CPSE+PBS mixture. Then, the mixture was incubated at 37°C for 10 minutes. In order to inhibit APC division, mitomycin C (0.5mg/ml) was added in a volume of 100 µL when there were 5x107 cells in 1mL of PBS and incubated at 37°C for 20 minutes. After filling 15 mL with pre-chilled medium and storing on ice for 5 minutes, it was washed twice with the culture medium and the cells were counted. Th17L+ cells, Th17L- cells, iTreg L+ cells and iTreg L- cells were added in a ratio of 2:1, 1:1, and 1:2 to CD4+ T cells, along with protein and anti-CD3 antibody (0.5ug/mL). After 3 days, the degree of inhibition of the proliferation of effector T cells was confirmed using CFSE-FL4, and the results are shown in Figure 1.

As shown in Figure 1, compared to the case where the expression level of Lrig1 is low, in Th17 cells with high Lrig1 expression levels, the effector T cell inhibitory effect was exhibited in all cases of 2:1, 1:1 and 1:2 ratios.

Having described specific parts of the present invention in detail above, it is clear to those skilled in the art that these specific techniques are only preferred embodiments, and that the scope of the present invention is not limited thereto. Accordingly, the substantial scope of the present invention will be defined by the appended claims and equivalents thereof.

### [Industrial Applicability]

The immune cells of the present invention have an activated inhibitory function, and the Lrig1 protein of activated immune cells can be used as a new target for immune-related diseases, cancer, or neurodegenerative or neuroinflammatory diseases.

## Claims

1. A Cell surface antigen of activated immune cells consisting of the Lrig1 protein or the gene encoding it.

2. The cell surface antigen of activated immune cells according to claim 1,
wherein the immune cells is at least one of those selected from the group consisting of T cells, B cells, NK cells, innate lymphoid cells, dendritic cells, neutrophils, eosinophils, basophils, macrophages, and mast cells.

3. A pharmaceutical composition comprising immune cell with the Lrig1 protein or the gene that encodes such protein as an active ingredient for the prevention or treatment of immune-related disease.

4. The pharmaceutical composition according to claim 3,
wherein the immune cell is at least one of those selected from the group consisting of T cells, B cells, NK cells, innate lymphoid cells, dendritic cells, neutrophils, eosinophils, basophils, macrophages, and mast cells.

5. The pharmaceutical composition according to claim 3,
wherein the immune-related disease is at least one of those selected from the group consisting of autoimmune disease, graft-versus-host disease, organ transplant rejection, asthma, atopy, or acute or chronic inflammatory disease.

6. The pharmaceutical composition according to claim 5,
wherein the autoimmune disease is at least one of those selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barre syndrome, chronic thyroiditis, multiple sclerosis, multiple myositis, ankylosing spondylitis, fibroblast and nodular multiple arteritis.

7. A pharmaceutical composition comprising immune cell with the Lrig1 protein or the gene that encodes such protein as an active ingredient for the prevention or treatment of cancer.

8. The pharmaceutical composition according to claim 7,
wherein the immune cell is at least one of those selected from the group consisting of T cells, B cells, NK cells, innate lymphoid cells, dendritic cells, neutrophils, eosinophils, basophils, macrophages, and mast cells.

9. The pharmaceutical composition according to claim 7,
wherein the cancer is at least one of those selected from the group consisting of gastric cancer, liver cancer, glioblastoma, ovarian cancer, colorectal cancer, head and neck cancer, bladder cancer, renal cell cancer, breast cancer, metastasis cancer, prostate cancer, pancreatic cancer, melanoma and lung cancer.

10. A pharmaceutical composition comprising immune cell with the Lrig1 protein or the gene that encodes such protein as an active ingredient for the prevention or treatment of neurodegenerative or neuroinflammatory diseases.

11. The pharmaceutical composition according to claim 10,
wherein the immune cell is at least one of those selected from the group consisting of T cells, B cells, NK cells, innate lymphoid cells, dendritic cells, neutrophils, eosinophils, basophils, macrophages, and mast cells.

12. The pharmaceutical composition according to claim 10,
wherein the neurodegenerative or neuroinflammatory disease is at least one of those selected from the group consisting of stroke, dementia, Alzheimer's disease, Parkinson's disease, Huntington's disease, Niemann-Pick disease, prion disease, Creutzfeldt-Jakob disease, frontotemporal dementia, Lewy dementia, Amyotrophic lateral sclerosis (ALS), paraneoplastic syndrome, corticobasal degeneration, multiple system atrophy disease, progressive supranuclear palsy, autoimmune disease of the nervous system, spinocerebellar ataxia, inflammatory and neuropathic pain, cerebrovascular disease, spinal cord injury, and tauopathy.

13. A diagnostic composition for immune-related diseases comprising an agent for measuring the expression levels of the Lrig1 protein on the surface of immune cells or of the gene that encodes such protein.

14. The diagnostic composition according to claim 13,
wherein the immune cell is at least one of those selected from the group consisting of T cells, B cells, NK cells, innate lymphoid cells, dendritic cells, neutrophils, eosinophils, basophils, macrophages, and mast cells.

15. A diagnostic kit for immune-related diseases comprising the diagnostic composition according to any one of claims 13 and 14.

16. A method for providing information for diagnosing immune-related diseases comprising measuring the expression level of Lrig1 protein or the gene that encodes such protein in immune cells among biological samples isolated from the objective individual.

17. A diagnostic composition for cancer comprising an agent for measuring the expression levels of the Lrig1 protein on the surface of immune cells or of the gene that encodes such protein.

18. The diagnostic composition according to claim 17,
wherein the immune cell is at least one of those selected from the group consisting of T cells, B cells, NK cells, innate lymphoid cells, dendritic cells, neutrophils, eosinophils, basophils, macrophages, and mast cells.

19. A diagnostic kit for cancer comprising the diagnostic composition according to any one of claims 17 and 18.

20. A method for providing information for diagnosing cancer comprising measuring the expression level of Lrig1 protein or the gene that encodes such protein in immune cells among biological samples isolated from the objective individual.

21. A diagnostic composition for neurodegenerative or neuroinflammatory diseases comprising an agent for measuring the expression levels of the Lrig1 protein on the surface of immune cells or of the gene that encodes such protein.

22. The diagnostic composition according to claim 21,
wherein the immune cell is at least one of those selected from the group consisting of T cells, B cells, NK cells, innate lymphoid cells, dendritic cells, neutrophils, eosinophils, basophils, macrophages, and mast cells.

23. A diagnostic kit for neurodegenerative or neuroinflammatory diseases comprising the diagnostic composition according to any one of claims 21 and 22.

24. A method for providing information for diagnosing neurodegenerative or neuroinflammatory diseases comprising measuring the expression level of Lrig1 protein or the gene that encodes such protein in immune cells among biological samples isolated from the objective individual.

25. A pharmaceutical composition that contains a bispecific antibody comprising an antibody or antigen-binding fragment thereof that specifically binds to Lrig1, and an antibody or antigen-binding fragment thereof that specially binds to immune cell surface markers as an active ingredient for preventing or treating immune-related diseases.

26. A pharmaceutical composition that comprises a bispecific antibody comprising an antibody or antigen-binding fragment thereof that specifically binds to Lrig1, and an antibody or antigen-binding fragment thereof that specially binds to immune cell surface markers as an active ingredient for preventing or treating cancer.

27. A pharmaceutical composition that comprises a bispecific antibody comprising an antibody or antigen-binding fragment thereof that specifically binds to Lrig1, and an antibody or antigen-binding fragment thereof that specially binds to immune cell surface markers as an active ingredient for preventing or treating neurodegenerative or neuroinflammatory diseases.

28. A pharmaceutical composition that comprises a bispecific antibodies or antigen-binding fragments that specifically bind to Lrig1 or immune cell surface markers and drug containing antibody-drug conjugates as an active ingredient for preventing or treating immune-related diseases.

29. A pharmaceutical composition that comprises a bispecific antibodies or antigen-binding fragments that specifically bind to Lrig1 or immune cell surface markers and drug containing antibody-drug conjugates as an active ingredient for preventing or treating cancer.

30. A pharmaceutical composition that comprises a bispecific antibodies or antigen-binding fragments that specifically bind to Lrig1 or immune cell surface markers and drug containing antibody-drug conjugates as an active ingredient for preventing or treating neurodegenerative or neuroinflammatory diseases.

31. A method for isolating activated immune cells comprising measuring the expression level of Lrig1 protein or the gene encoding it in a separate biological sample.

32. A method of culturing activated immune cells comprising isolating activated immune cells by measuring the expression level of Lrig1 protein or the gene encoding it in a separate biological sample and culturing such activated immune cells.

33. A method for screening cell therapies for immune-related diseases comprising measuring the expression level of Lrig1 protein or the gene encoding it in a separate biological sample.

34. A method for screening cell therapies for cancer comprising measuring the expression level of Lrig1 protein or the gene encoding it in a separate biological sample.

35. A method for screening cell therapies for neurodegenerative or neuroinflammatory diseases comprising measuring the expression level of Lrig1 protein or the gene encoding it in a separate biological sample.
